# EUROPÄISCHE PATENTSCHRIFT

(11) **EP 3 164 699 B1**
(45) Veröffentlichungstag und Bekanntmachung des Hinweises auf die Patenterteilung: **28.07.2021**
(21) Anmeldenummer: 16713724.9
(22) Anmeldetag: 11.03.2016
(51) Int. Cl.: G01N 27/22, B65H 63/06, G01N 27/24, G01N 33/36

(54) **KAPAZITIVE SENSORBAUGRUPPE FÜR EIN TEXTILES PRÜFGERÄT**
CAPACITIVE SENSOR ASSEMBLY FOR A TEXTILE TESTING DEVICE
MODULE CAPTEUR CAPACITIF POUR UN APPAREIL DE CONTRÔLE DE TEXTILE

(30) Priorität: 20.03.2015 CH 4132015
(43) Veröffentlichungstag der Anmeldung: 10.05.2017
(73) Patentinhaber: Uster Technologies AG, 8610 Uster (CH)
(72) Erfinder: JOSS, Rolf, 8810 Horgen (CH); PFÄFFLI, Fabian, 8308 Illnau (CH); DE VRIES, Loris, 8625 Gossau (CH); ZIGANEK, Norbert, 8605 Gutenswil (CH)
(74) Vertreter: Pliska, Pavel
(86) Internationale Anmeldenummer: PCT/CH2016/000047
(87) Internationale Veröffentlichungsnummer: WO 2016/149847

(56) Entgegenhaltungen:
- EP-A2- 2 700 936
- CH-A5- 563 021
- CH-A5- 589 287
- US-A- 2 604 513

## Beschreibung

### FACHGEBIET

Die vorliegende Erfindung liegt auf dem Gebiet der textilen Qualitätsprüfung und bezieht sich auf eine kapazitive Sensorbaugruppe für ein Prüfgerät zur Prüfung von länglichem textilem Prüfgut wie Garn, Vorgarn oder Faserband, gemäss dem Oberbegriff des ersten Patentanspruchs. Die Erfindung betrifft auch ein Prüfgerät zur Prüfung von länglichem textilem Prüfgut wie Garn, Vorgarn oder Faserband sowie ein Verfahren zur Prüfung von länglichem textilem Prüfgut wie Garn, Vorgarn oder Faserband mittels einer kapazitiven Sensorbaugruppe, gemäss den Oberbegriffen von weiteren Patentansprüchen.

### STAND DER TECHNIK

Vorrichtungen zur kapazitiven Untersuchung eines länglichen textilen Prüfgutes, wie z. B. eines Garns, eines Vorgarns oder eines Faserbandes, sind bekannt. Sie beinhalten einen Messspalt, durch den das Prüfgut in seiner Längsrichtung bewegt wird. Der Messspalt wird durch zwei zum Prüfgut parallele und beidseits des Prüfgutes angeordnete Flächen gebildet, denen Elektroden eines Messkondensators zugeordnet sind. Aufgrund von Messungen der Kapazität des Kondensators können Aussagen über Masse und/oder Material des im Kondensator befindlichen Prüfgutes gemacht werden.

Die US-6,072,319 A offenbart eine kapazitive Vorrichtung zum Messen von Eigenschaften eines textilen Produktes in einem Messspalt, in dem das textile Produkt eingeführt wird. Die Vorrichtung weist einen Messschaltkreis und einen Kompensationsschaltkreis auf. Mit dem letzteren lassen sich Störeinflüsse wie Feuchtigkeitsänderungen der im Messspalt befindlichen Luft oder ungewollte mechanische Änderungen der Messspaltbreite kompensieren. Der Messschaltkreis beinhaltet einen Messkondensator, dessen ebene Elektroden den Messspalt zur Aufnahme des Prüfgutes bilden, und Leitungen zu den Elektroden. Der Kompensationsschaltkreis beinhaltet einen ähnlich aufgebauten Kompensationskondensator und entsprechende Leitungen. Die US-6,072,319 A zeigt auch eine Anordnung von mehreren Messspalten verschiedener Breite, die in einem einzigen Element gemeinsam angeordnet sind. Die Messspalte haben unterschiedliche Breiten, damit Produkte mit unterschiedlichen Durchmessern ausgemessen werden können.

Die GB-1'476'203 A (und die CH-563'021 A5 aus derselben Patentfamilie) befasst sich mit dem Problem von Umwelteinflüssen, welche die Messresultate von textilen Prüfgeräten verfälschen können. Sie stellt fest, dass textile Messkondensatoren äusserst empfindlich auf instabile Temperaturverhältnisse im Inneren und Äusseren des Prüfgerätes sind. Temperaturgradienten verformen Komponenten der Messkondensatoren. Ausserdem verfälschen auch Ablagerungen an den Messkondensatoren die Messresultate. Zur Erhöhung der Langzeitstabilität des Prüfgerätes sieht die GB-1'476'203 A einen Luftstrom vor, der die Kondensatorplatten der Messkondensatoren kühlt und Ablagerungen verhindert.

Die US-3,879,660 A (und die CH-589'287 A5 aus derselben Patentfamilie) schlägt vor, den gesamten kapazitiven Messfühlerkopf über normale Umgebungstemperatur hinaus zu erwärmen, um eine grössere Stabilität hinsichtlich Temperatur- und Feuchtigkeitsänderungen zu erreichen.

Das Problem der Verfälschung der textilen Messresultate durch Temperaturinstabilitäten hat sich bei modernen textilen Prüfgeräten noch verschärft. Kapazitive Sensorbaugruppen von modernen Prüfgeräten beinhalten komplexe elektronische Schaltungen zum Ansteuern der Messkondensatoren und zum Auswerten ihrer Ausgangssignale. Solche elektronischen Schaltungen sind mit vielen elektronischen Bauelementen wie Halbleiterverstärkern bestückt, die zusammen eine grosse Verlustwärme erzeugen. Beim Einschalten des Prüfgerätes führt diese Verlustwärme zu einer langsamen Erwärmung der Sensorbaugruppe und damit zu einer thermischen Drift, welche die Messresultate bis zum Erreichen eines thermischen Gleichgewichtszustandes verfälscht. Eine Belüftung der kapazitiven Sensorbaugruppe schafft nur teilweise Abhilfe, weil die zur Belüftung verwendete Aussenluft auch Temperaturänderungen unterworfen ist, welche ihrerseits die Messresultate verfälschen.

### DARSTELLUNG DER ERFINDUNG

Es ist eine Aufgabe der vorliegenden Erfindung, eine kapazitive Sensorbaugruppe für ein textiles Prüfgerät zur Verfügung zu stellen, die weitgehend stabil und ohne oder mit nur kleiner Temperaturdrift misst. Eine weitere Aufgabe ist es, ein Prüfgerät zur Prüfung von länglichem textilem Prüfgut wie Garn, Vorgarn oder Faserband zur Verfügung zu stellen, das ebenfalls weitgehend stabil und ohne Temperaturdrift misst. Eine noch weitere Aufgabe ist es, ein Verfahren zur Prüfung von länglichem textilem Prüfgut wie Garn, Vorgarn oder Faserband mittels einer kapazitiven Sensorbaugruppe anzugeben, das weitgehend stabile Messresultate ohne Temperaturdrift liefert.

Diese und andere Aufgaben werden durch die im ersten Patentanspruch definierte kapazitive Sensorbaugruppe gelöst. Weitere Patentansprüche geben ein Prüfgerät zur Prüfung von länglichem textilem Prüfgut sowie ein Verfahren zur Prüfung von länglichem textilem Prüfgut an. Vorteilhafte Ausführungsformen sind in den abhängigen Patentansprüchen angegeben.

Die Erfindung beruht auf der Idee, einen Temperatursensor und einen Temperaturaktor für mindestens einen Teil der kapazitiven Sensorbaugruppe zur Verfügung zu stellen. So kann einerseits eine Temperatur des mindestens einen Teils gemessen und andererseits der mindestens eine Teil je nach Bedarf gekühlt oder geheizt werden. Dies ermöglicht es auch, die Temperatur des mindestens einen Teils aktiv zu regeln. Besonders vorteilhaft ist es, die Temperatur wärmeempfindliche Bauteile der kapazitiven Sensorbaugruppe zu stabilisieren. Aber auch die Abführung der Verlustwärme von Bauteilen der kapazitiven Sensorbaugruppe kann Vorteile bringen.

Die erfindungsgemässe kapazitive Sensorbaugruppe ist für ein Prüfgerät zur Prüfung von länglichem textilem Prüfgut wie Garn, Vorgarn oder Faserband bestimmt. Sie beinhaltet mindestens eine Messelektrode als Teil eines Messkondensators zur kapazitiven Prüfung des Prüfgutes und eine mit der mindestens einen Messelektrode elektrisch verbundene elektronische Schaltung. Ferner beinhaltet die kapazitive Sensorbaugruppe mindestens einen Temperatursensor zur Messung einer Temperatur mindestens eines Teils der kapazitiven Sensorbaugruppe und einen ansteuerbaren, mit dem mindestens einen Teil der kapazitiven Sensorbaugruppe thermisch verbundenen elektrothermischen Wandler.

"Thermisch verbunden" heisst in dieser Schrift, dass zwischen den thermisch verbundenen Elementen eine gute Wärmeleitung möglich sein soll. Zu diesem Zweck stehen die Elemente entweder in direktem Kontakt miteinander oder sind über ein Medium oder mehrere Medien miteinander verbunden, die einen guten Wärmedurchgang erlauben, d. h. nicht thermisch isolieren. Der Wärmedurchgangskoeffizient zwischen den beiden Elementen sollte grösser als ca. 200 W/(m²·K) und vorzugsweise grösser als ca. 1000 W/(m²·K) sein.

In einer Ausführungsform ist der elektrothermische Wandler mit der elektronischen Schaltung thermisch verbunden. Bevorzugt ist der elektrothermische Wandler mit folgenden Arten von Bauelementen der elektronischen Schaltung thermisch verbunden:
- Bauelemente, welche die Temperaturempfindlichkeit eines Ausgangssignals der elektronischen Schaltung stark beeinflussen. Darunter werden in der vorliegenden Schrift vorzugsweise solche Bauelemente verstanden, deren Temperaturänderung um 1 °C eine Änderung des Ausgangssignals der elektronischen Schaltung um mindestens 3 mV und vorzugsweise um mindestens 10 mV verursachen. Der Fachmann ist in der Lage, solche Bauelemente zumindest empirisch zu ermitteln, indem er ein Bauelement nach dem anderen lokal erwärmt, dabei die Temperaturänderung des Bauelementes misst und gleichzeitig die Änderung des Ausgangssignals der elektronischen Schaltung misst.
- Bauelemente, welche im Betrieb eine grosse Verlustwärme abgeben. Darunter werden in der vorliegenden Schrift vorzugsweise solche Bauelemente verstanden, die im Betrieb eine Verlustwärme von mindestens 10 mW und vorzugsweise von mindestens 100 mW abgeben. Verlustwärmen von elektronischen Bauelementen können üblicherweise den betreffenden Datenblättern entnommen werden.

Die elektronische Schaltung kann auf einer Leiterplatte angeordnet sein.

Der elektrothermische Wandler ist vorzugsweise als flächiges Peltier-Element ausgebildet.

In einer Ausführungsform ist die elektronische Schaltung auf einer Leiterplatte angeordnet. Die thermische Verbindung durch eine mit einer Fläche des Peltier-Elementes einerseits und mit einer Fläche der Leiterplatte andererseits in grossflächigem Kontakt stehende wärmeleitende flächige Füllschicht hergestellt. "Grossflächiger Kontakt" heisst in diesem Zusammenhang, dass der Kontakt nicht nur punktuell ist, sondern auf einem beträchtlichen Bruchteil, z. B. mindestens der Hälfte, der Fläche des Peltier-Elementes und der Fläche der Leiterplatte hergestellt ist. Die Füllschicht besteht vorzugsweise aus einem glasfaserverstärkten Polymer. Eine von der Leiterplatte abgewandte Fläche des Peltier-Elementes kann direkt oder über einen Kühlkörper mit Luft kühlbar sein.

In einer Ausführungsform ist der mindestens eine Temperatursensor ein Teil der elektronischen Schaltung. Er ist vorzugsweise in der Nähe folgender Arten von Bauelementen der elektronischen Schaltung angeordnet:
- Bauelemente, welche die Temperaturempfindlichkeit eines Ausgangssignals der elektronischen Schaltung stark beeinflussen. Darunter werden in der vorliegenden Schrift vorzugsweise solche Bauelemente verstanden, deren Temperaturänderung um 1 °C eine Änderung des Ausgangssignals der elektronischen Schaltung um mindestens 3 mV und vorzugsweise um mindestens 10 mV verursachen.
- Bauelemente, welche im Betrieb eine grosse Verlustwärme abgeben. Darunter werden in der vorliegenden Schrift vorzugsweise solche Bauelemente verstanden, die im Betrieb eine Verlustwärme von mindestens 10 mW und vorzugsweise von mindestens 100 mW abgeben.

"In der Nähe angeordnet" heisst in diesem Zusammenhang so angeordnet, dass der Temperatursensor so gut wie möglich eine Temperatur des betreffenden Bauelementes misst. Der Temperatursensor ist vorzugsweise auf einer Leiterplatte unmittelbar neben dem betreffenden Bauelement angeordnet.

In einer Ausführungsform beinhaltet die kapazitive Sensorbaugruppe mehrere Messelektroden, die als ebene Plattenelektroden ausgebildet sind und jeweils paarweise einen Plattenkondensator als Messkondensator bilden.

Das erfindungsgemässe Prüfgerät dient zur Prüfung von länglichem textilem Prüfgut wie Garn, Vorgarn oder Faserband. Es weist eine erfindungsgemässe kapazitive Sensorbaugruppe und einen Regelkreis zur Regelung der Temperatur des mindestens einen Teils der kapazitiven Sensorbaugruppe auf einen Temperatursollwert auf. Der Regelkreis beinhaltet einen Regler, dem der Temperatursollwert als Führungsgrösse vorgebbar ist, den mindestens einen Temperatursensor, dessen Ausgangssignal als Regelgrösse dem Regler zuführbar ist, und den elektrothermischen Wandler als Stellglied, dem ein vom Regler ausgegebenes Stellsignal zuführbar ist.

Im erfindungsgemässen Verfahren zur Prüfung von länglichem textilem Prüfgut wie Garn, Vorgarn oder Faserband mittels einer kapazitiven Sensorbaugruppe wird eine Referenztemperatur gemessen. Ein Temperatursollwert wird in Abhängigkeit von der gemessenen Referenztemperatur bestimmt. Danach wird laufend eine Temperatur mindestens eines Teils der kapazitiven Sensorbaugruppe gemessen und mit einem von dem Temperatursollwert und der gemessenen Temperatur abhängigen Stellsignal auf den Temperatursollwert regelt, während die Prüfung des länglichen textilen Prüfgutes mittels der kapazitiven Sensorbaugruppe stattfindet.

In einer Ausführungsform wird das Stellsignal laufend mit einem vorgegebenen Grenzwert verglichen und der Temperatursollwert in Abhängigkeit von der gemessenen Referenztemperatur neu bestimmt, wenn das Stellsignal den Grenzwert überschreitet.

In einer Ausführungsform des erfindungsgemässen Verfahrens ist die kapazitive Sensorbaugruppe ein Teil eines erfindungsgemässen Prüfgerätes. Die gemessene Referenztemperatur kann eine im Prüfgerät herrschende Innentemperatur und/oder eine in einer Umgebung des Prüfgerätes herrschende Aussentemperatur sein.

Offenbart wird ferner eine Verwendung, welche nicht Teil der beanspruchten Erfindung ist, eines ansteuerbaren elektrothermischen Wandlers als Stellglied zur Regelung einer Temperatur mindestens eines Teils einer kapazitiven Sensorbaugruppe für ein Prüfgerät zur Prüfung von länglichem textilem Prüfgut wie Garn, Vorgarn oder Faserband.

Dank der Erfindung wird die Temperatur der kapazitiven Sensorbaugruppe weitgehend stabilisiert, unabhängig davon, ob in der kapazitiven Sensorbaugruppe selbst oder in einer Umgebung der kapazitiven Sensorbaugruppe Wärme erzeugt oder abgeführt wird. Dies erlaubt Messungen unter konstanten thermischen Bedingungen, ohne Temperaturdrift.

### AUFZÄHLUNG DER ZEICHNUNGEN

Nachfolgend werden Ausführungsformen der Erfindung anhand der Zeichnungen detailliert erläutert.
- Figur 1: zeigt ein erfindungsgemässes Prüfgerät in einer perspektivischen Ansicht.
- Figur 2: zeigt eine erfindungsgemässe kapazitive Sensorbaugruppe in einer Explosionsansicht.
- Figur 3: zeigt einen Teil der Sensorbaugruppe von Figur 2, und zwar (a) in einer Explosionsansicht und (b) in einer perspektivischen Ansicht.
- Figur 4: zeigt ein Flussdiagramm eines erfindungsgemässen Verfahrens zur Prüfung von länglichem textilem Prüfgut.
- Figur 5: zeigt, gegen die Zeit aufgetragen, (a) ein gemessenes Ausgangssignal eines Messkondensators und (b) eine gemessene Temperatur in einer Umgebung des Prüfgerätes für eine erfindungsgemässe Sensorbaugruppe.
- Figur 6: zeigt, gegen die Zeit aufgetragen, (a) ein gemessenes Ausgangssignal eines Messkondensators und (b) eine gemessene Temperatur in einer Umgebung des Prüfgerätes für eine Sensorbaugruppe gemäss dem Stand der Technik.

### AUSFÜHRUNG DER ERFINDUNG

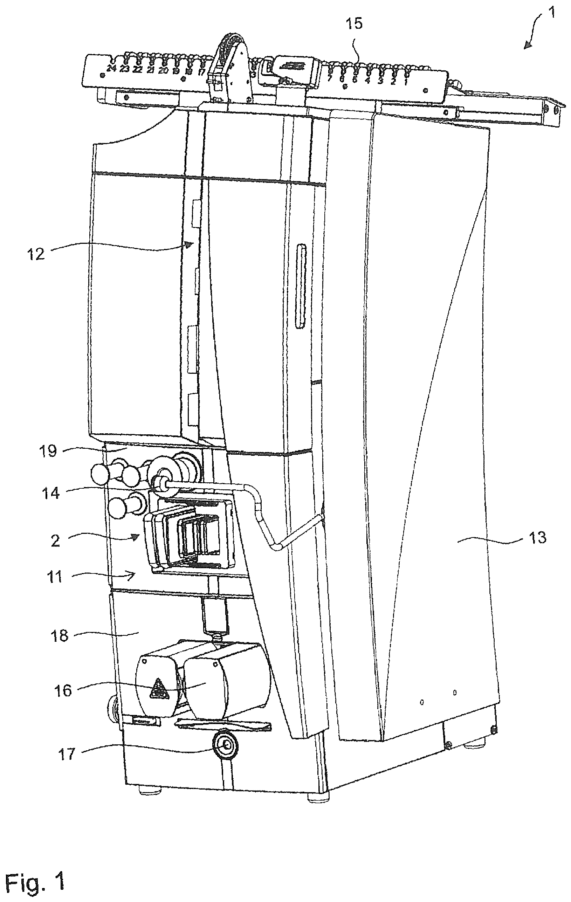

**Figur 1** zeigt beispielhaft eine Ausführungsform des erfindungsgemässen Prüfgerätes 1 in einer Ansicht von links vorn. Das Prüfgerät 1 weist eine Front 11 auf, entlang welcher ein Prüfgutpfad 12 für ein zu prüfendes (nicht eingezeichnetes) textiles Prüfgut verläuft. Auf einer Seite des Prüfgerätes 1, welche von der Front 11 verschieden ist, ist eine automatische Einführungsvorrichtung 13 zum Einführen des Prüfgutes in den Prüfgutpfad 12 angebracht. Zum Einbringen in den Prüfgutpfad 12 wird das Prüfgut von einem verschiebbaren und drehbaren Greifer 14 der Einführungsvorrichtung 13 ergriffen und durch eine entsprechende Bewegung des Greifers 14 eingebracht.

Während des Prüfvorgangs tritt das Prüfgut durch eine automatische Garnwechselvorrichtung 15 in den Prüfgutpfad 12 ein. Im Prüfgutpfad 12 durchläuft das Prüfgut verschiedene Sensoren. Das Prüfgut wird von einer Fördereinrichtung 16 entlang seiner Längsrichtung durch den Prüfgutpfad 12 gefördert. Die Fördereinrichtung 16 kann z. B. als Rollenlieferwerk mit zwei zusammenwirkenden Förderrollen von denen mindestens eine zur Rotation angetrieben ist, ausgebildet sein. Schliesslich verlässt das Prüfgut den Prüfgutpfad 12 durch eine Absaugöffnung 17.

Das Prüfgerät 1 hat einen turmartigen Aufbau. Der turmartige Aufbau kann verschiedene Funktionsmodule umfassen. Ein erstes Funktionsmodul 18 beinhaltet im vorliegenden Fall die Fördereinrichtung 16 und die Absaugöffnung 17. Ein zweites Funktionsmodul 19 beinhaltet eine kapazitive Sensorbaugruppe 2 zur Gleichmässigkeitsprüfung des Prüfgutes. Weitere, oberhalb des zweiten Funktionsmoduls 19 befindliche Funktionsmodule können weitere, z. B. optische Sensoren beinhalten, sind jedoch wegen einer Frontabdeckung in Figur 1 nicht sichtbar.

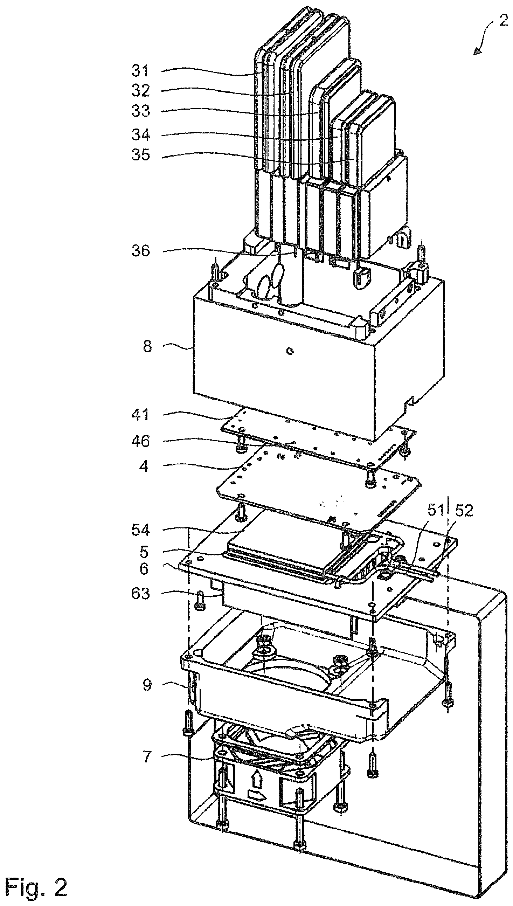

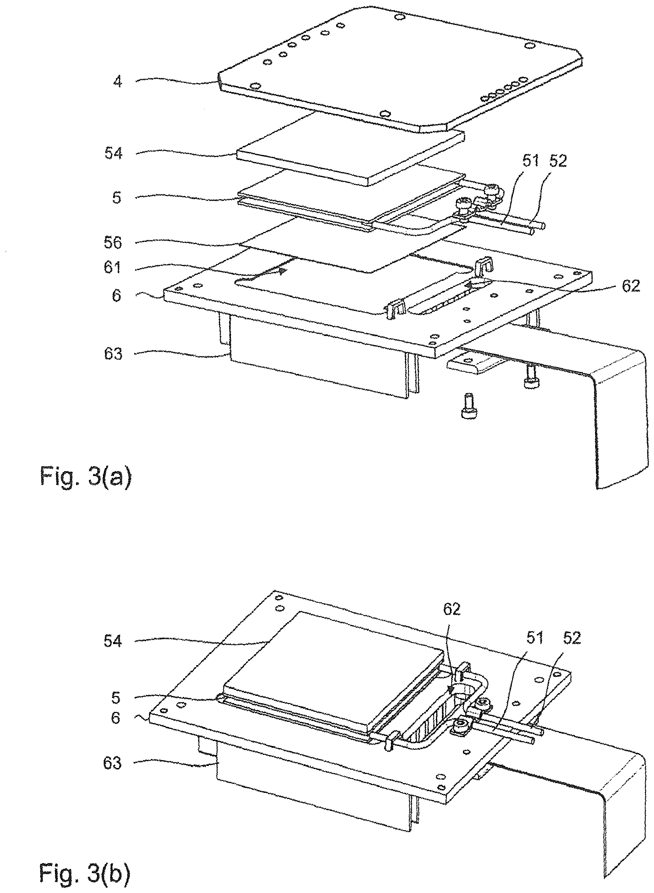

**Figur 2** zeigt die erfindungsgemässe kapazitive Sensorbaugruppe 2 in einer Explosionsansicht, und **Figur 3** zeigt einen Teil davon (a) in einer Explosionsansicht und (b) in einer perspektivischen Ansicht, jedoch ohne Leiterplatte.

In der hier diskutierten beispielhaften Ausführungsform beinhaltet die Sensorbaugruppe 2 fünf ebene, starre Substrate 31-35. Die Substrate 31-35 sind parallel zueinander angeordnet und bilden so vier dazwischen liegende Durchgangsöffnungen oder Messspalte. Ein (nicht eingezeichnetes) längliches textiles Prüfgut wie Garn, Vorgarn oder Faserband kann durch einen der Messspalte geführt und durch diesen entlang seiner Längsachse bewegt werden. Die Messspalte weisen unterschiedliche Breiten auf, so dass das Prüfgut je nach seinem Querschnitt in einem Messspalt mit geeigneter Breite ausgemessen werden kann. Die Breiten der Messspalte können z. B. im Bereich zwischen 0.1 mm und 10 mm liegen.

Zwei benachbarte Substrate 31, 32 tragen jeweils in oder auf ihren einander zugewandten Seitenwänden mindestens eine (in den Zeichnungen nicht sichtbare) Messelektrode. Zwei einander gegenüber liegende Messelektroden bilden zusammen mit dem dazwischen liegenden Messspalt einen Messkondensator zur kapazitiven Prüfung des Prüfgutes. Ausser den Messelektroden können die Substrate 31-35 weitere (in den Zeichnungen nicht sichtbare) elektronische Bauelemente tragen, insbesondere Kompensationselektroden von Kompensationskondensatoren und elektrische Leitungen zum Verbinden der Messelektroden und der Kompensationselektroden mit einer elektronischen Schaltung. Einzelheiten einer möglichen Ausgestaltung der Substrate 31-35 und der darauf befindlichen elektronischen Bauelemente können der US-6,072,319 A entnommen werden.

Die mindestens eine Messelektrode ist mit einer (in den Zeichnungen nicht sichtbaren) elektronischen Schaltung elektrisch verbundenen, die sich im vorliegenden Ausführungsbeispiel auf einer Leiterplatte 4 befindet. Die elektronische Schaltung auf der Leiterplatte 4 hat hauptsächlich zwei Aufgaben. Erstens steuert sie die Messkondensatoren an, stellt ihnen also die entsprechenden elektrischen Wechselspannungen zur Verfügung. Zweitens wertet sie die Ausgangssignale der Messkondensatoren aus. Sie ist mit vielen elektronischen Bauelementen wie Halbleiterverstärkern bestückt, die zusammen eine grosse Verlustwärme erzeugen.

Zwischen der Leiterplatte 4 und den Substraten 31-35 kann eine Zwischenleiterplatte 41 angebracht sein, die (in den Zeichnungen nicht sichtbare) elektronische Bauelemente zur Vorverarbeitung von Messsignalen enthalten kann, bspw. Vorverstärker. Die Zwischenleiterplatte 41 ist mit den elektronischen Bauelementen auf den Substraten 31-35 einerseits und mit der Leiterplatte 4 andererseits elektrisch verbunden. Die Ebenen der Leiterplatte 4 und der Zwischenleiterplatte 41 liegen parallel zueinander, und die Ebenen der Substrate 31-35 stehen senkrecht dazu. Die Substrate 31-35 weisen mit den darauf befindlichen elektronischen Bauelementen elektrisch verbundene Steckkontakte 36 auf, die in entsprechende Buchsen 46 auf der Zwischenleiterplatte 41 gesteckt sind.

Um Verfälschungen der Messresultate durch Temperaturdrift zu vermeiden, wird die Temperatur der Leiterplatte 4 geregelt. Zu diesem Zweck ist die Leiterplatte 4 über eine wärmeleitende flächige Füllschicht 54 mit einem elektrothermischen Wandler 5 thermisch verbunden. In der hier diskutierten Ausführungsform ist der elektrothermische Wandler 5 als flächiges Peltier-Element ausgeführt. Es kann z. B. ein Peltier-Element vom Typ TEC1-12712T125 des Herstellers Beijing Huimao Cooling Equipment Co., Ltd., Peking (China) verwendet werden, welches die Masse 62 mm × 62 mm × 4.3 mm hat. Das Peltier-Element 5 kann über elektrische Leitungen 51, 52 angesteuert werden.

Die wärmeleitende flächige Füllschicht 54 sorgt für eine gute thermische Verbindung zwischen der Leiterplatte 4 und dem Peltier-Element 5. Einerseits bedeckt sie einen möglichst grossen Teil einer dem Peltier-Element 5 zugewandten Fläche der Leiterplatte 4 und vorteilhafterweise diejenigen Bauelemente der elektronischen Schaltung, die besonders temperaturempfindlich sind und/oder die im Betrieb eine besonders grosse Verlustwärme abgeben. Beispiele für solche Bauelemente sind Operationsverstärker, insbesondere in einer Vorverstärkerstufe und in einer Endstufe, sowie MOSFET-Transistoren, die für eine Signalverarbeitung eingesetzt werden. Andererseits bedeckt die Füllschicht 54 einen möglichst grossen Teil einer ihr zugewandten, kalten Fläche des Peltier-Elementes 5. Die Füllschicht 54 besteht z. B. aus einem glasfaserverstärkten Polymer. Es kann z. B. eine Füllschicht vom Typ Gap Pad® 5000S35 des Herstellers The Bergquist Company, Chanhassen, MN (USA) verwendet werden. Diese hat die Masse 62 mm × 62 mm × 2 mm und eine Wärmeleitfähigkeit von λ = 5.0 W/(m·K). Bei Vernachlässigung der Wärmeübergangskoeffizienten ergibt sich somit für diese Füllschicht ein Wärmedurchgangskoeffizient von k ≈ λ/d = 2500 W/(m²·K).

Eine von der Leiterplatte 4 abgewandte Fläche des Peltier-Elementes 5 ist mittels eines wärmeleitenden doppelseitigen Klebebandes 56 auf einem metallischen Kühlkörper 6 befestigt. In Figur 3 sind im Kühlkörper 6 eine Aussparung 61 für das doppelseitige Klebeband 56 und eine Durchgangsöffnung 62 für ein (nicht eingezeichnetes) Kabel zur elektrischen Verbindung der Leiterplatte 4 mit weiteren Elementen des Prüfgerätes 1 sichtbar. Der Kühlkörper 6 weist auf einer vom Peltier-Element 5 abgewandten Seite Kühlrippen 63 auf. Diese werden von einem Ventilator 7 belüftet. So wird die von der Leiterplatte 4 abgewandte, warme Fläche des Peltier-Elementes 5 mit Luft gekühlt. Dabei wird die in der elektronischen Schaltung erzeugte Wärme wirksam von der Sensorbaugruppe 2 abgeführt, so dass sich die Sensorbaugruppe 2 im Betrieb nicht wesentlich erwärmt.

Die der Zwischenleiterplatte 41 zugewandten Enden der Substrate 31-35, die Zwischenleiterplatte 41, die Leiterplatte 5, die Füllschicht 54, das Peltier-Element 5 und das doppelseitige Klebeband 56 sind vorzugsweise in einem metallischen ersten Gehäuseteil 8 untergebracht. Dieser dient erstens der Befestigung der Substrate 31-35 und zweitens als mechanischer Schutz vor äusseren Einflüssen. Drittens schützt der erste Gehäuseteil 8 die empfindlichen elektronischen Bauteile und die darin bearbeiteten elektrischen Signale als Faradayscher Käfig vor äusseren elektromagnetischen Störungen. Die Kühlrippen 63 und der Ventilator 7 können in einem zweiten Gehäuseteil 9 untergebracht sein. Der Kühlkörper 6 sowie der erste Gehäuseteil 8 und zweite Gehäuseteil 9, die am Kühlkörper 6 befestigt sind, bilden zusammen ein Gehäuse der Sensorbaugruppe 2.

Die Temperatur der Leiterplatte 4 wird in einem Regelkreis gemäss einem Regelverfahren geregelt, die nachfolgend beschrieben sind.

Das erfindungsgemässe Prüfgerät 1 weist einen (in den Zeichnungen nicht dargestellten) Referenztemperatursensor auf, der eine im Prüfgerät 1 herrschende Innentemperatur und/oder eine in einer Umgebung des Prüfgerätes 1 herrschende Aussentemperatur misst. Ferner beinhaltet das erfindungsgemässe Prüfgerät 1 einen (in den Zeichnungen nicht dargestellten) Regler.

Die erfindungsgemässe Sensorbaugruppe 2 beinhaltet einen (in den Zeichnungen nicht dargestellten) Temperatursensor, dessen Ausgangssignal als Regelgrösse für die Regelung der Temperatur der Leiterplatte 4 dem Regler zuführbar ist. Der Temperatursensor ist vorzugsweise als Teil der elektronischen Schaltung auf der Leiterplatte 4, bspw. als oberflächenmontiertes Bauelement (surface-mounted device, SMD), ausgebildet. Besonders vorteilhaft ist es, den Temperatursensor in der Nähe eines solchen Bauelementes der elektronischen Schaltung, das eine grosse Temperaturempfindlichkeit aufweist, bspw. eines Endstufenverstärkers, anzuordnen.

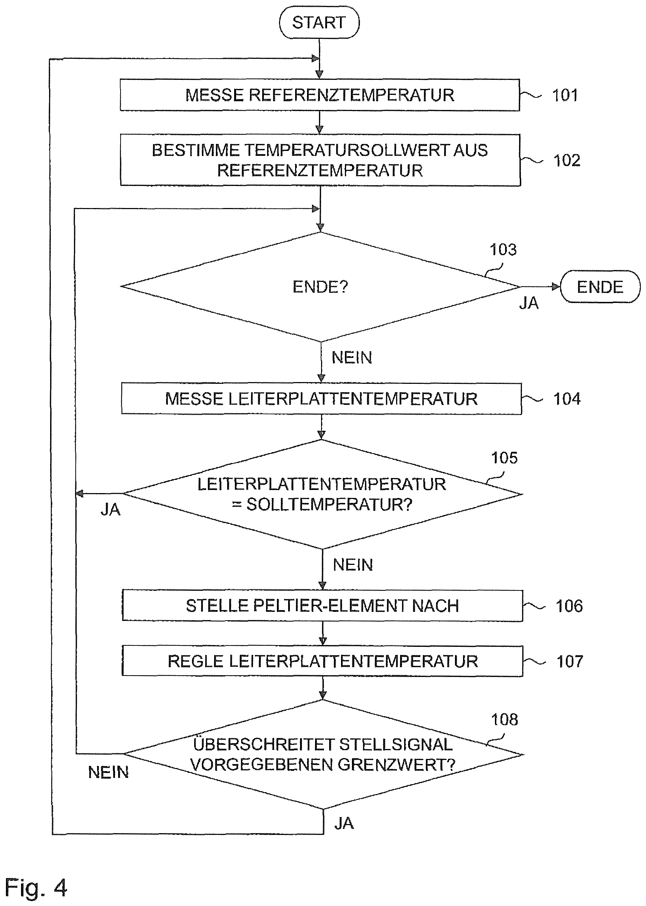

Ein Flussdiagramm einer Ausführungsform des erfindungsgemässen Verfahrens, die im vorliegenden Beispiel anhand des oben diskutierten Prüfgerätes 1 ausgeführt wird, ist in **Figur 4** gezeichnet. Auf einen entsprechenden Befehl hin, bspw. kurz nach dem Einschalten des Prüfgerätes 1, misst 101 der Referenztemperatursensor eine Referenztemperatur. Die Referenztemperatur kann z. B. eine im Prüfgerät 1 herrschende Innentemperatur und/oder eine in einer Umgebung des Prüfgerätes 1 herrschende Aussentemperatur sein. Aus der gemessenen Referenztemperatur bestimmt 102 das Prüfgerät 1 automatisch einen Temperatursollwert für die Leiterplatte 4 oder einen anderen Teil der kapazitiven Sensorbaugruppe 2. Der Temperatursollwert liegt vorzugsweise etwas höher, bspw. um 1 °C höher, als die gemessene Referenztemperatur. Er dient als Führungsgrösse für den Regelkreis und wird vorzugsweise im Regler gespeichert.

Der Temperatursensor auf der Leiterplatte 4 misst 104 laufend die Temperatur der Leiterplatte 4. Der Regler vergleicht 105 laufend die vom Temperatursensor gemessene Temperatur mit dem Temperatursollwert. Stimmen die beiden Temperaturwerte überein, so braucht es keinen Regeleingriff. Sind sie jedoch voneinander verschieden, so berechnet der Regler ein Stellsignal und führt es dem Peltier-Element 5 zu, um es nachzustellen 106. Das Peltier-Element 5 dient als Stellglied des Regelkreises. Aufgrund des vom Regler erhaltenen Stellsignals regelt es 107 die Temperatur der Leiterplatte 4.

Um eine Überlastung des Regelkreises zu verhindern, ist ein Kontrollmechanismus vorgesehen. Dieser kontrolliert 108 laufend die Last des Reglers und passt den Temperatursollwert an, wenn die Last zu hoch ist. Dies ist dann der Fall, wenn das vom Regler dem Peltier-Element 5 zugeführte Stellsignal einen vorgegebenen Grenzwert überschreitet. Dann wird eine neue Referenztemperatur gemessen 101 und der Messwert zur Bestimmung 102 eines neuen Temperatursollwertes verwendet. Mit dem neuen Temperatursollwert wird dann der Regler an einem optimaleren Arbeitspunkt betrieben.

Auf einen entsprechenden Befehl hin, bspw. beim Ausschalten des Prüfgerätes 1, wird das Verfahren beendet 103.

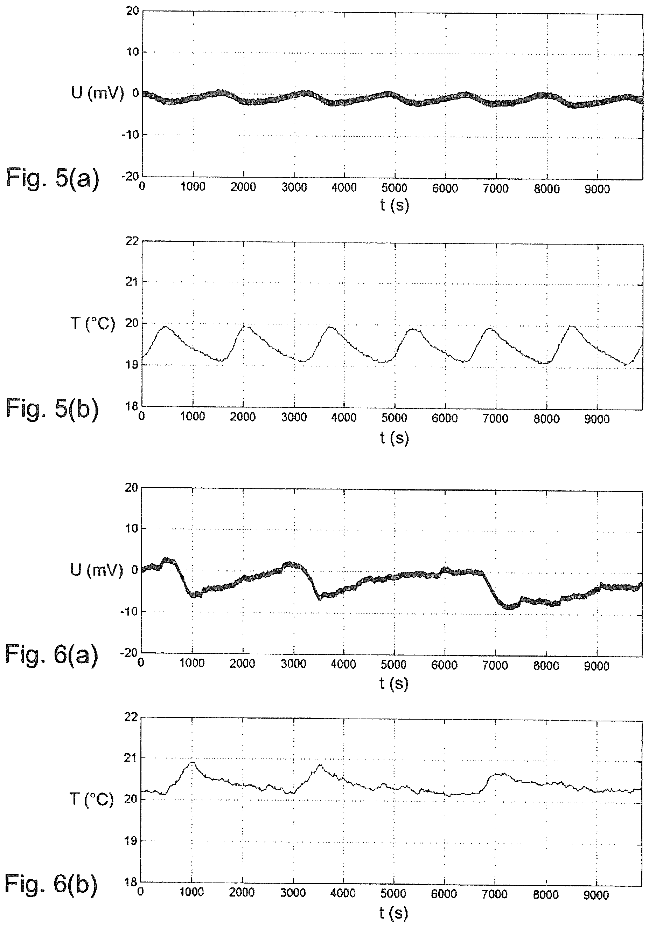

Die **Figuren 5 und 6** erlauben einen Vergleich zwischen der erfindungsgemässen kapazitiven Sensorbaugruppe 2 **(****Figur 5****)** und einer kapazitiven Sensorbaugruppe gemäss dem Stand der Technik **(****Figur 6****)**. Es handelt sich um Messresultate aus realen Versuchen, in denen ohne Prüfgut über einen Zeitraum von ca. 2 ¾ Stunden untersucht wurde, wie Ausgangssignale von Messkondensatoren von der Umgebungstemperatur abhängen. Im unteren Teil (b) der Figuren ist jeweils ein zeitlicher Verlauf einer Umgebungstemperatur T(t) des Prüfgerätes 1 aufgezeichnet. Die Temperatur T(t) veränderte sich mit der Zeit t. Im oberen Teil (a) der Figuren ist jeweils der entsprechende zeitliche Verlauf eines Ausgangssignals U(t) eines Messkondensators aufgezeichnet. Die Ausgangssignale U(t) der Messkondensatoren wurden in beiden Versuchen von der Umgebungstemperatur T(t) in unerwünschter Weise beeinflusst und somit verfälscht, jedoch unterschiedlich stark. Die folgende Tabelle listet die gemessenen maximalen Signalhübe ΔU und maximalen Temperaturhübe ΔT sowie die daraus berechneten Temperaturempfindlichkeiten ΔU/ΔT auf:

| **Versuch** | **Maximaler Signalhub ΔU (mV)** | **Maximaler Temperaturhub ΔT (°C)** | **Temperaturempfindlichkeit ΔU/ΔT (mV/°C)** |
|---|---|---|---|
| Fig. 5: Erfindung | 3.51 | 0.96 | 3.6 |
| Fig. 6: Stand der Technik | 12.66 | 0.80 | 15.8 |

Der Vergleich zeigt, dass die Temperaturempfindlichkeit ΔU/ΔT bei der erfindungsgemässen Sensorbaugruppe 2 (Figur 5) ca. dreimal kleiner ist als bei der Sensorbaugruppe gemäss dem Stand der Technik (Figur 6). Diese Versuchsresultate belegen die Wirksamkeit und Nützlichkeit der Erfindung.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören.

### BEZUGSZEICHENLISTE

- 1: Prüfgerät
- 11: Front
- 12: Prüfgutpfad
- 13: Einführungsvorrichtung
- 14: Greifer
- 15: Garnwechselvorrichtung
- 16: Fördereinrichtung
- 17: Absaugöffnung
- 18: erstes Funktionsmodul
- 19: zweites Funktionsmodul

- 2: kapazitive Sensorbaugruppe

- 31-35: Substrate
- 36: Steckkontakte

- 4: Leiterplatte
- 41: Zwischenleiterplatte
- 46: Buchsen

- 5: elektrothermischer Wandler
- 51, 52: elektrische Leitungen
- 54: wärmeleitende flächige Füllschicht
- 56: wärmeleitendes doppelseitiges Klebeband

- 6: Kühlkörper
- 61: Aussparung
- 62: Durchgangsöffnung
- 63: Kühlrippen

- 7: Ventilator

- 8: erster Gehäuseteil

- 9: zweiter Gehäuseteil

## Patentansprüche

1. Kapazitive Sensorbaugruppe (2) für ein Prüfgerät (1) zur Prüfung von länglichem textilem Prüfgut wie Garn, Vorgarn oder Faserband, mit
mindestens einer Messelektrode als Teil eines Messkondensators zur kapazitiven Prüfung des Prüfgutes und
einer mit der mindestens einen Messelektrode elektrisch verbundenen elektronischen Schaltung,
**gekennzeichnet durch**
mindestens einen Temperatursensor zur Messung einer Temperatur mindestens eines Teils (4) der kapazitiven Sensorbaugruppe (2) und
einen ansteuerbaren, mit dem mindestens einen Teil (4) der kapazitiven Sensorbaugruppe (2) thermisch verbundenen elektrothermischen Wandler (5).

2. Kapazitive Sensorbaugruppe (2) nach Anspruch 1, wobei der elektrothermische Wandler (5) mit der elektronischen Schaltung thermisch verbunden ist.

3. Kapazitive Sensorbaugruppe (2) nach Anspruch 2, wobei der elektrothermische Wandler (5) mit mindestens einem solchen Bauelement der elektronischen Schaltung, dessen Temperaturänderung um 1 °C eine Änderung eines Ausgangssignals der elektronischen Schaltung um mindestens 3 mV und vorzugsweise um mindestens 10 mV verursacht, thermisch verbunden ist.

4. Kapazitive Sensorbaugruppe (2) nach Anspruch 2 oder 3, wobei der elektrothermische Wandler (5) mit mindestens einem solchen Bauelement der elektronischen Schaltung, das im Betrieb eine Verlustwärme von mindestens 10 mW und vorzugsweise von mindestens 100 mW abgibt, thermisch verbunden ist.

5. Kapazitive Sensorbaugruppe (2) nach einem der vorangehenden Ansprüche, wobei der elektrothermische Wandler (5) als flächiges Peltier-Element ausgebildet ist.

6. Kapazitive Sensorbaugruppe (2) nach Anspruch 5, wobei die elektronische Schaltung auf einer Leiterplatte (4) angeordnet ist und die thermische Verbindung durch eine mit einer Fläche des Peltier-Elementes (5) einerseits und mit einer Fläche der Leiterplatte (4) andererseits in grossflächigem Kontakt stehende wärmeleitende flächige Füllschicht (54) hergestellt ist.

7. Kapazitive Sensorbaugruppe (2) nach Anspruch 6, wobei die Füllschicht (54) aus einem glasfaserverstärkten Polymer besteht.

8. Kapazitive Sensorbaugruppe (2) nach Anspruch 6 oder 7, wobei eine von der Leiterplatte (4) abgewandte Fläche des Peltier-Elementes (5) direkt oder über einen Kühlkörper (6) mit Luft kühlbar ist.

9. Kapazitive Sensorbaugruppe (2) nach einem der vorangehenden Ansprüche, wobei der mindestens eine Temperatursensor ein Teil der elektronischen Schaltung ist.

10. Kapazitive Sensorbaugruppe (2) nach Anspruch 9, wobei der mindestens eine Temperatursensor in der Nähe eines solchen Bauelementes der elektronischen Schaltung, dessen Temperaturänderung um 1 °C eine Änderung eines Ausgangssignals der elektronischen Schaltung um mindestens 3 mV und vorzugsweise um mindestens 10 mV verursacht, und/oder das im Betrieb eine Verlustwärme von mindestens 10 mW und vorzugsweise von mindestens 100 mW abgibt, angeordnet ist.

11. Kapazitive Sensorbaugruppe (2) nach einem der vorangehenden Ansprüche, wobei die kapazitive Sensorbaugruppe (2) mehrere Messelektroden beinhaltet, die als ebene Plattenelektroden ausgebildet sind und jeweils paarweise einen Plattenkondensator als Messkondensator bilden.

12. Prüfgerät (1) zur Prüfung von länglichem textilem Prüfgut wie Garn, Vorgarn oder Faserband,
**gekennzeichnet durch**
eine kapazitive Sensorbaugruppe (2) nach einem der vorangehenden Ansprüche und einen Regelkreis zur Regelung der Temperatur des mindestens einen Teils (4) der kapazitiven Sensorbaugruppe (2) auf einen Temperatursollwert, welcher Regelkreis
einen Regler, dem der Temperatursollwert als Führungsgrösse vorgebbar ist, den mindestens einen Temperatursensor, dessen Ausgangssignal als Regelgrösse dem Regler zuführbar ist, und
den elektrothermischen Wandler (5) als Stellglied, dem ein vom Regler ausgegebenes Stellsignal zuführbar ist,
beinhaltet.

13. Verfahren zur Prüfung von länglichem textilem Prüfgut wie Garn, Vorgarn oder Faserband mittels einer kapazitiven Sensorbaugruppe (2),
**dadurch gekennzeichnet, dass**
eine Referenztemperatur gemessen wird (101),
ein Temperatursollwert in Abhängigkeit von der gemessenen Referenztemperatur bestimmt (102) wird und danach
laufend eine Temperatur mindestens eines Teils (4) der kapazitiven Sensorbaugruppe (2) gemessen (104) und mit einem von dem Temperatursollwert und der gemessenen Temperatur abhängigen Stellsignal auf den Temperatursollwert regelt wird (107), während die Prüfung des länglichen textilen Prüfgutes mittels der kapazitiven Sensorbaugruppe (2) stattfindet.

14. Verfahren nach Anspruch 13, wobei
das Stellsignal laufend mit einem vorgegebenen Grenzwert verglichen wird (108) und
der Temperatursollwert in Abhängigkeit von der gemessenen (101) Referenztemperatur neu bestimmt wird (102), wenn das Stellsignal den Grenzwert überschreitet.

15. Verfahren nach Anspruch 13 oder 14, wobei die kapazitive Sensorbaugruppe (2) ein Teil eines Prüfgerätes (1) nach Anspruch 12 ist.

16. Verfahren nach Anspruch 15, wobei die gemessene Referenztemperatur eine im Prüfgerät (1) herrschende Innentemperatur und/oder eine in einer Umgebung des Prüfgerätes (1) herrschende Aussentemperatur ist.

## Claims

1. A capacitive sensor assembly (2) for a testing device (1) for testing elongated textile test material such as yarn, roving or sliver, comprising
at least one measuring electrode as a part of a measuring capacitor for the capacitive testing of the test material, and
an electronic circuit which is electrically connected to the at least one measuring electrode,
**characterized by**
at least one temperature sensor for measuring a temperature of at least one part (4) of the capacitive sensor assembly (2) and
a controllable electrothermal converter (5) which is thermally connected to the at least one part (4) of the capacitive sensor assembly (2).

2. A capacitive sensor assembly (2) according to claim 1, wherein the electrothermal converter (5) is thermally connected to the electronic circuit.

3. A capacitive sensor assembly (2) according to claim 2, wherein the electrothermal converter (5) is thermally connected to at least one such component of the electronic circuit whose change in temperature by 1°C produces a change of an output signal of the electronic circuit by at least 3 mV and preferably by at least 10 mV.

4. A capacitive sensor assembly (2) according to claim 2 or 3, wherein the electrothermal converter (5) is thermally connected to at least one such component of the electronic circuit which in operation emits heat losses of at least 10 mW and preferably at least 100 mW.

5. A capacitive sensor assembly (2) according to one of the preceding claims, wherein the electrothermal converter (5) is formed as a flat Peltier element.

6. A capacitive sensor assembly (2) according to claim 5, wherein the electronic circuit is arranged on a printed circuit board (4), and the thermal connection is produced by a thermally conductive, flat filling layer (54) which is in contact over a great area with a surface of the Peltier element (5) on the one hand and a surface of the printed circuit board (4) on the other hand.

7. A capacitive sensor assembly (2) according to claim 6, wherein the filling layer (54) consists of a glass-fiber-reinforced polymer.

8. A capacitive sensor assembly (2) according to claim 6 or 7, wherein a surface of the Peltier element (5) which faces away from the printed circuit board (4) is coolable with air directly or via a heat sink (6).

9. A capacitive sensor assembly (2) according to one of the preceding claims, wherein the at least one temperature sensor is part of the electronic circuit.

10. A capacitive sensor assembly (2) according to claim 9, wherein the at least one temperature sensor is arranged close to such a component of the electronic circuit whose change in temperature by 1°C produces a change of an output signal of the electronic circuit by at least 3 mV and preferably by at least 10 mV, and/or which in operation emits heat losses of at least 10 mW and preferably at least 100 mW.

11. A capacitive sensor assembly (2) according to one of the preceding claims, wherein the capacitive sensor assembly (2) contains several measuring electrodes, which are formed as flat plate electrodes and each form in pairs a plate capacitor as a measuring capacitor.

12. A testing device (1) for testing elongated textile test material such as yarn, roving or sliver,
**characterized by**
a capacitive sensor assembly (2) according to one of the preceding claims, and
a control circuit for controlling the temperature of the at least one part (4) of the capacitive sensor assembly (2) to a temperature setpoint, which control circuit contains
a controller to which the temperature setpoint is predeterminable as a reference variable,
the at least one temperature sensor whose output signal is suppliable as a control parameter to the controller, and
the electrothermal converter (5) as an actuator which is suppliable with an actuating signal that is output by the controller.

13. A method for testing elongated textile test material such as yarn, roving or sliver by means of a capacitive sensor assembly (2),
**characterized in that**
a reference temperature is measured (101),
a temperature setpoint is determined (102) depending on the measured reference temperature, and thereafter
a temperature of at least one part (4) of the capacitive sensor assembly (2) is measured (104) continuously and is controlled (107) to the temperature setpoint by an actuating signal dependent on the temperature setpoint and the measured temperature while the testing of the elongated textile test material occurs by means of the capacitive sensor assembly (2).

14. A method according to claim 13, wherein
the actuating signal is compared continuously with a predetermined threshold value (108) and
the temperature setpoint is newly determined (102) depending on the measured (101) reference temperature when the actuating signal exceeds the threshold value.

15. A method according to claim 13 or 14, wherein the capacitive sensor assembly (2) is a part of a testing device (1) according to claim 12.

16. A method according to claim 15, wherein the measured reference temperature is an internal temperature prevailing in the testing device (1) and/or an external temperature prevailing in ambient environment of the testing device (1).

## Revendications

1. Module capteur capacitif (2) pour un appareil de contrôle (1) destiné à contrôler un échantillon textile allongé tel qu'un fil, une mèche ou un ruban de fibres, comportant au moins une électrode de mesure faisant partie d'un condensateur de mesure pour le contrôle capacitif de l'échantillon et
un circuit électronique relié électriquement à ladite au moins une électrode de mesure,
**caractérisé par**
au moins un capteur de température pour mesurer une température d'au moins une partie (4) du module capteur capacitif (2) et
un transducteur électrothermique commandable (5) relié thermiquement à ladite au moins une partie (4) du module capteur capacitif (2).

2. Module capteur capacitif (2) selon la revendication 1, dans lequel le transducteur électrothermique (5) est relié thermiquement au circuit électronique.

3. Module capteur capacitif (2) selon la revendication 2, dans lequel le transducteur électrothermique (5) est relié thermiquement à au moins un composant du circuit électronique dont une variation de température de 1 °C provoque une variation d'un signal de sortie du circuit électronique d'au moins 3 mV et de préférence d'au moins 10 mV.

4. Module capteur capacitif (2) selon la revendication 2 ou 3, dans lequel le transducteur électrothermique (5) est relié thermiquement à au moins un composant du circuit électronique qui présente une perte de chaleur d'au moins 10 mW et de préférence d'au moins 100 mW en fonctionnement.

5. Module capteur capacitif (2) selon l'une des revendications précédentes, dans lequel le transducteur électrothermique (5) est réalisé sous la forme d'un élément Peltier plan.

6. Module capteur capacitif (2) selon la revendication 5, dans lequel le circuit électronique est disposé sur une carte de circuit imprimé (4) et la liaison thermique est produite par une couche de remplissage plane thermoconductrice (54) qui est en contact à grande surface avec une surface de l'élément Peltier (5) d'une part et avec une surface de la carte de circuit imprimé (4) d'autre part.

7. Module capteur capacitif (2) selon la revendication 6, dans lequel la couche de remplissage (54) est constituée d'un polymère renforcé par des fibres de verre.

8. Module capteur capacitif (2) selon la revendication 6 ou 7, dans lequel une surface de l'élément Peltier (5) opposée à la carte de circuit imprimé (4) peut être refroidie avec de l'air directement ou par l'intermédiaire d'un dissipateur thermique (6).

9. Module capteur capacitif (2) selon l'une des revendications précédentes, dans lequel ledit au moins un capteur de température fait partie du circuit électronique.

10. Module capteur capacitif (2) selon la revendication 9, dans lequel ledit au moins un capteur de température est disposé à proximité d'un composant du circuit électronique dont une variation de température de 1 °C provoque une variation d'un signal de sortie du circuit électronique d'au moins 3 mV et de préférence d'au moins 10 mV, et/ou qui présente une perte de chaleur d'au moins 10 mW et de préférence d'au moins 100 mW en fonctionnement.

11. Module capteur capacitif (2) selon l'une des revendications précédentes, dans lequel le module capteur capacitif (2) comprend plusieurs électrodes de mesure qui sont réalisées sous la forme d'électrodes planes et forment chaque fois par paires un condensateur à plaques servant de condensateur de mesure.

12. Appareil de contrôle (1) destiné à contrôler un échantillon textile allongé tel qu'un fil, une mèche ou un ruban de fibres,
**caractérisé par**
un module capteur capacitif (2) selon l'une des revendications précédentes et
un circuit de régulation pour réguler la température de ladite au moins une partie (4) du module capteur capacitif (2) sur une valeur de consigne de température, lequel circuit de régulation comprend :
un régulateur auquel la valeur de consigne de température peut être prédéfinie en tant que grandeur de commande,
ledit au moins un capteur de température dont le signal de sortie peut être amené au régulateur en tant que grandeur régulée et
le transducteur électrothermique (5) en tant qu'actionneur auquel un signal de réglage délivré par le régulateur peut être amené.

13. Procédé pour contrôler un échantillon textile allongé tel qu'un fil, une mèche ou un ruban de fibres au moyen d'un module capteur capacitif (2),
**caractérisé en ce que**
une température de référence est mesurée (101),
une valeur de consigne de température est déterminée (102) en fonction de la température de référence mesurée et ensuite
une température d'au moins une partie (4) du module capteur capacitif (2) est mesurée en continu (104) et régulée (107) sur la valeur de consigne de température au moyen d'un signal de réglage qui dépend de la valeur de consigne de température et de la température mesurée, pendant que le contrôle de l'échantillon textile allongé a lieu au moyen du module capteur capacitif (2).

14. Procédé selon la revendication 13, dans lequel
le signal de réglage est comparé en continu (108) avec une valeur limite prédéfinie et la valeur de consigne de température est déterminée à nouveau (102) en fonction de la température de référence mesurée (101) lorsque le signal de réglage dépasse la valeur limite.

15. Procédé selon la revendication 13 ou 14, dans lequel le module capteur capacitif (2) fait partie d'un appareil de contrôle (1) selon la revendication 12.

16. Procédé selon la revendication 15, dans lequel la température de référence mesurée est une température intérieure régnant dans l'appareil de contrôle (1) et/ou une température extérieure régnant dans un environnement de l'appareil de contrôle (1).
